# EUROPÄISCHE PATENTSCHRIFT

(11) **EP 0 804 405 B1**
(45) Veröffentlichungstag und Bekanntmachung des Hinweises auf die Patenterteilung: **07.04.1999**
(21) Anmeldenummer: 96900560.2
(22) Anmeldetag: 04.01.1996
(51) Int. Cl.: C07C 53/19, C07B 57/00, C07C 51/09, C07C 51/487

(54) **VERFAHREN ZUR HERSTELLUNG VON OPTISCH AKTIVEN 2-HALOGENPROPIONSÄUREN**
PROCESS FOR PRODUCING OPTICALLY ACTIVE 2-HALOGEN PROPIONIC ACIDS
PROCEDE DE PREPARATION DE 2-HALOACIDES PROPIONIQUES A ACTIVITE OPTIQUE

(30) Priorität: 19.01.1995 DE 19501452
(43) Veröffentlichungstag der Anmeldung: 05.11.1997
(73) Patentinhaber: BASF AKTIENGESELLSCHAFT, 67056 Ludwigshafen (DE)
(72) Erfinder: MERKLE, Hans, Rupert, D-67061 Ludwigshafen (DE); FRETSCHNER, Erich, D-69239 Neckarsteinach (DE); HANSEN, Hanspeter, D-67061 Ludwigshafen (DE); ZIPPERER, Bernhard, D-67246 Dirmstein (DE)
(86) Internationale Anmeldenummer: EP9600012
(87) Internationale Veröffentlichungsnummer: WO9622272

(56) Entgegenhaltungen:
- WO-A-93/22269
- FR-A- 2 701 706

## Beschreibung

Die vorliegende Erfindung betrifft ein neues Verfahren zur Herstellung von optisch aktiven 2-Halogenpropionsäuren.

Es ist bekannt, L-2-Chlorpropionsäure und deren Natriumsalz durch alkalische Hydrolyse von L-2-Chlorpropionsäureisobutylester herzustellen. Hierbei fällt jedoch oft ein qualitativ minderwertiges Produkt an (ca. 90 bis 95 % Enantiomerenuberschuß), das zudem noch 5 bis 10 % Milchsäure als Nebenprodukt enthält.

FR 27 00 164 beschreibt die Synthese von L-2-Chlorpropionsäureestern durch Reaktion von o-silylierten Ketenacetalen mit N-Chlorsuccinimid und deren Hydrolyse mit Alkalimetallhydroperoxiden zu L-2-Chlorpropionsäure.

Aus JP 57 094 295, JP 62 205 797, EP-A-196 625, JP 61 111 699 und Appl. Biochem. Biotechnol. 9 (3), (1984) 255 ist bekannt, racemische 2-Chlorpropionsäureester mittels Enzymen enantioselektiv zu spalten. Nachteilig hierbei ist allerdings neben der maximal möglichen Ausbeute von lediglich 50 % - bezogen auf das Racemat - die ungenügende Enantiomerenreinheit der gebildeten 2-Chlorpropionate.

Ebenso ist die Racematspaltung von 2-Halogencarbonsäureestern durch Lipase aus Candida Cylindracea bekannt (J. Am. Chem. Soc. 107, 7072 (1985)).

Nach Bio. Med. Chem. Lett. 1991 I, 339, erhält man optisch aktive Phenoxypropionsäureester durch enzymatische Veresterung der entsprechenden racemischen Säuren, wobei je nach Enzym eines der Enantiomeren verestert wird und das andere als Säure zurückbleibt. Die Trennung der optisch aktiven Verbindungen erfolgt dann durch Destillation und/oder Extraktion. Je nach Verfahrensbedingungen erhält man Anteile der D- oder L-Isomeren von 70 % bis ca. 95 %.

Bei enzymatischer Dehalogenierung racemischer Chlorpropionsäure, wird das D-Enantiomer in die Milchsäure übergeführt und die L-Chlorpropionsäure bleibt unverändert. Die maximal möglichen Ausbeuten liegen bei 50 % - bezogen auf das Racemat - (Biocatalysis, D.A. Abramowicz CZ (Ed) van Nostrand R. New York (1990), Journal of General Microbiology 128, 1755 (1982)).

Ferner wird in der EP-A-257 716 ein kontinuierliches Verfahren dargestellt, bei dem D,L-2-Brompropionsäuremethylester als Racemat eingesetzt und in einem 2-Phasensystem mittels Candida cylindracea Lipase in L-(-)-2-Brompropionsäuremethylester umgewandelt wird. Bei diesem Verfahren werden jedoch keine Hydrolyseprodukte isoliert.

Des weiteren beschreiben S. K. Dahod und P. Sinta-Mangano in Biotechnol. Bioeng. 30 (8), 995 (1987) die Lipase-katalysierte Hydrolyse von L-2-Chlorpropionsäuremethylester in Gegenwart von Kohlenstofftetrachlorid. Nachteilig hierbei ist neben der Verwendung eines chlorierten Lösungsmittels die geringe Ausbeute von ca. 30 % bei nur 95 % Enantiomerenreinheit.

Außerdem ist der EP-A-511 526 ein Verfahren zur enzymatischen Hydrolyse von racemischen 2-Chlorpropionsäureestern in einem 2-Phasensystem, das aus Wasser und einem mit Wasser weitgehend unmischbaren organischen Lösungsmittel für den Ester besteht, zu entnehmen. Dieses Verfahren ist sehr aufwendig, denn die organische Phase muß mehrmals abgetrennt, mit der Hydrolase in Kontakt gebracht und wieder rückgeführt werden. Da trotz relativ langer Reaktionszeiten nur ein unvollständiger Umsatz stattfindet und keine enantiomerenreinen Produkte erhalten werden, ist diese Methode für die technische Herstellung von L-2-Chlorpropionsäure und deren Natriumsalz ungeeignet.

Weitere Verfahren zur Herstellung von optisch aktiven 2-Halogencarbonsäuren sind Racematspaltungen von 2-Halogencarbonsäureamiden mit Mikroorganismen, z.B. Pseudomones (DE 4 117 255 Al) und 2-Halogencarbonsäurenitrilen durch Mikroorganismen (Pure and Appl. Chem. 62, 1441 (1990)).

Aufgabe der vorliegenden Erfindung war es daher, ein einfaches Verfahren zur Herstellung von optisch aktiven 2-Halogenpropionsäuren mit möglichst hoher chemischer und optischer Reinheit (mindestens ca. 98 % Enantiomerenüberschuß) zu entwickeln.

Demgemäß wurde ein Verfahren zur Herstellung von optisch aktiven 2-Halogenpropionsäuren gefunden, welches dadurch gekennzeichnet ist, daß man die entsprechenden optisch aktiven 2-Halogenpropionsäurealkylester bei erhöhter Temperatur mit einer Carbonsäure in einer Umacylierungsreaktion unter Bildung der optisch aktiven 2-Halogenpropionsäure und des Carbonsäurealkylesters umsetzt und die erhaltene optisch aktive 2-Halogenpropionsäure aus dem Reaktionsgemisch abtrennt.

Das erfindungsgemäße Verfahren wird anhand der nachfolgenden Reaktionsgleichung beschrieben, 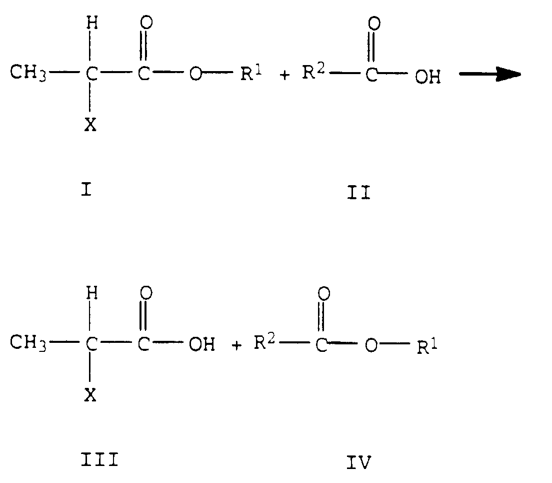 worin I der optisch aktive 2-Halogenpropionsäurealkylester mit X in der Bedeutung von Halogen, II die Carbonsäure, III die erhaltene optisch aktive 2-Halogenpropionsäure und IV der bei der Umacylierung gebildete Carbonsäurealkylester ist.

Im allgemeinen bedeutet X in den optisch aktiven 2-Halogenpropionsäurealkylestern I und 2-Halogenpropionsäuren III Chlor, Brom, Jod, vorzugsweise Chlor, Brom, insbesondere Chlor.

In der vorstehend angegebenen Reaktionsgleichung bedeutet R¹ im allgemeinen C₁-C₈-Alkyl, vorzugsweise C₁-C₆-Alkyl, R² bedeutet im allgemeinen Wasserstoff oder C₁-C₄-Alkyl.

Zweckmäßig werden als Carbonsäuren II Ameisensäure, Essigsäure, Propionsäure, vorzugsweise Ameisensäure oder Essigsäure, insbesondere Ameisensäure verwendet.

Die Herstellung der als Ausgangsprodukt zu verwendenden optisch aktiven 2-Halogenpropionsäurealkylester erfolgt vorteilhaft ausgehend von den entsprechenden optisch aktiven Milchsäureestern durch deren Halogenierung. Beispielsweise erfolgt die Herstellung von L-2-Chlorpropionsäurealkylestern durch Halogenierung von den entsprechenden D-Milchsäurealkylestern durch Austausch der Hydroxylgruppe gegen Chlor. Die D-Milchsäurealkylester können auf bekannte Weise chloriert werden (vgl. z.B. EP-A-401 104, JP 61 057 534 (1986), JP 02 104 560 (1990), JP 61 068 445 (1986) und FR-A-24 59 221). Die Chlorierung erfolgt vorzugsweise mit Thionylchlorid in Gegenwart eines Katalysators, z.B. N,N-Dimethylformamid, wobei Inversion am asymmetrischen C-Atom erfolgt. Das Rohprodukt wird von den Schwersiedern abgetrennt und anschließend destillativ gereinigt.

Nach der vorstehend beschriebenen Weise kann z.B. der L-2-Chlorpropionsäureisobutylester mit einer chemischen Reinheit von ca. 98 bis 99,8 % erhalten werden. Die optische Reinheit (L:D) beträgt etwa 98:2 bis 100:0 %.

Zur erfindungsgemäßen Umacylierung können die optisch aktiven 2-Halogenpropionsäurealkylester aber auch ohne vorherige Reinigung eingesetzt werden.

Die Umsetzung zur Herstellung der optisch aktiven 2-Halogenpropionsäuren erfolgt zweckmäßig bei Temperaturen von 40°C bis 200°C, bevorzugt von 80°C bis 150°C, besonders vorteilhaft am Siedepunkt der zur Umacylierung eingesetzten Carbonsäure bzw. am Siedepunkt des bei der Umacylierung gebildeten Carbonsäurealkylesters.

Die Reaktion kann bei Normaldruck sowie bei Unterdruck oder Überdruck durchgeführt werden. Im allgemeinen arbeitet man im Druckbereich von 0,1 bis 10 bar, vorzugsweise 0,5 bis 3 bar.

Die für die Umacylierung einzusetzende Carbonsäure kann in der zum eingesetzten 2-Halogenpropionsäurealkylester stöchiometrischen Menge verwendet werden. Vorzugsweise wird die Carbonsaure jedoch im Überschuß verwendet, der im allgemeinen 10 % bis 5000 %, bevorzugt 200 % bis 1000 % beträgt.

Die Carbonsäure kann mit dem 2-Halogenpropionsäurealkylester vorgelegt werden oder auch während der Umacylierungsreaktion zugefügt werden bzw. teilweise vorgelegt und der Rest während der Umacylierungsreaktion zugefügt werden.

Es kann vorteilhaft sein, die Umsetzung in Anwesenheit eines sauren Katalysators durchzuführen. Geeignete saure Katalysatoren sind z.B. Mineralsäuren wie Phosphorsäure, Halogenwasserstoffsauren, Schwefelsäure, organische Sulfonsäuren wie p-Toluolsulfonsäure, Ionenaustauscher in der Wasserstofform, Lewissäuren. Besonders geeignet ist Schwefelsäure. Im allgemeinen werden die Katalysatoren in Mengen von 0,1 Gew.-% bis 10 Gew.-%, bezogen auf den 2-Halogenpropionsäurealkylester, eingesetzt.

Es kann weiter vorteilhaft sein, während der Umacylierungsreaktion geringe Mengen Wasser, im allgemeinen in Mengen von 0 Gew.-% bis 50 Gew.-%, vorzugsweise 0,01 Gew.-% bis 30 Gew.-%, bezogen auf den 2-Halogenpropionsäurealkylester, zuzufügen, wobei bei der Verwendung von Ameisensäure als Carbonsäure die Wassermenge geringer ausfallen oder ganz entfallen kann, da Ameisensäure sich unter den Reaktionsbedingungen zum Teil unter Wasserbildung zersetzt. Die überwiegende Wassermenge wird in der Regel während der destillativen Entfernung des entstandenen Carbonsäurealkylesters mit abgetrennt.

In der Regel arbeitet man ohne zusätzliches Lösungsmittel, doch können auch unter den Reaktionsbedingungen inerte Lösungsmittel, z.B. chlorierte Kohlenwasserstoffe, aliphatische Kohlenwasserstoffe wie Hexan, oder aromatische Kohlenwasserstoffe wie Toluol, Xylol als Verdünnungsmittel eingesetzt werden.

Es kann vorteilhaft sein, den bei der Umacylierung gebildeten Carbonsäurealkylester während der Umsetzung, zweckmäßig durch Destillation, in dem Maße, wie er gebildet wird, abzutrennen.

Die Aufarbeitung des Reaktionsgemisches zur Gewinnung der optisch aktiven 2-Halogenpropionsäure erfolgt in der Regel durch fraktionierte Destillation. Dies wird beispielsweise in der Weise durchgeführt, daß man aus dem Reaktionsgemisch den bei der Umacylierung gebildeten Carbonsäurealkylester und gegebenenfalls noch vorhandene Carbonsäure durch Abdestillieren abtrennt und die optisch aktive 2-Halogenpropionsäure als Rückstand erhält. Das als Rückstand erhaltene Rohprodukt kann dann durch weitere Reinigung, zweckmäßig durch Destillation, als Reinprodukt erhalten werden.

Die vorstehend beschriebenen Destillationen können bei Normaldruck, im Vakuum oder bei Überdruck durchgeführt werden. Falls die Umacylierung unter Verwendung von sauren Katalysatoren durchgeführt wird, werden die sauren Katalysatoren zweckmäßig vor der Destillation abgetrennt oder neutralisiert, z.B. durch Zugabe von Alkali- oder Erdalkalihydroxiden oder von Alkalisalzen schwächerer Säuren, z.B. Alkaliformiate und Alkaliacetate wie Natriumformiat und Natriumacetat.

Bei der erfindungsgemäßen Umacylierung erhält man die optisch aktiven 2-Halogenpropionsäuren auf sehr wirtschaftliche Weise in hoher Ausbeute und hoher optischer Reinheit.

Bei Synthese und Aufarbeitung bleibt die optische Reinheit erhalten oder verringert sich nur geringfügig. Die optisch aktiven 2-Chlorpropionsäuren sind Einsatzstoffe bei Synthesen von Pharmaka und Pflanzenschutzmitteln, z.B. bei Synthesen von optisch aktiven Aryloxy-Propionsäuren wie D-2,4-Dichlorphenoxypropionsäure, D-2-Methyl-4-chlorphenoxypropionsäure, Butyl-2-(4-[5-(trifluormethyl)-2-pyridinyl]oxy]phenoxy]-propionat (Fluazifopbutyl), Ethyl-2-[4-[(6-chlor-2-benzoxazolyl)oxy]phenoxy]propionat (Fenoxaprop-ethyl).

Die nachfolgenden Beispiele veranschaulichen die Erfindung.

### Beispiel 1

493,5 g (3,0 mol) L-2-Chlorpropionsäureisobutylester (D: 0,8 % L: 99,2 %) und 138 g (3,0 mol) Ameisensäure (100%ig) werden mit 5,6 g (0,057 mol) Schwefelsäure (100%ig) im Kolben vorgelegt. Innerhalb von 3 Stunden wird eine Lösung aus 552 g (12 mol) Ameisensäure (100%ig) und 5,6 g (0,057 mol) Schwefelsäure (100%ig) bei Rückfluß (112°C) zudosiert. Über eine Kolonne wird gleichzeitig ein Azeotrop bestehend aus Isobutylformiat, Ameisensäure und Wasser aus dem Reaktionskolben abdestilliert. Nach Zulaufende wird die Reaktionslösung noch weitere 2 Stunden bei Siedetemperatur nachgerührt. Unter Abdestillieren des erwähnten Azeotrops steigt die Temperatur auf 115°C an. Der Reaktionskolbeninhalt wird mit 15,5 g (0,228 mol) Natriumformiat neutralisiert und anschließend fraktioniert destilliert. Man erhält 308,6 g L-2-Chlorpropionsäure (D: 0,8 % L: 99,2 %) mit einem Gehalt von 99,8 % (GC) Kp₄₀: 102°C; das entspricht einer Ausbeute von 94,6 % d. Th.

### Beispiel 2

493,5 g (3,0 mol) L-2-Chlorpropionsäureisobutylester (D: 0,8 % L: 99,2 %) und 138 g (3,0 mol) Ameisensäure (100%ig) werden mit 5,6 g (0,057 mol) Schwefelsäure (100%ig) im Kolben vorgelegt. Innerhalb von 3 Stunden wird eine Lösung aus 306,7 g (6 mol) Ameisensäure (90%ig) und 5,6 g (0,057 mol) Schwefelsäure (100%ig) bei Rückfluß (112°C) zudosiert. Über eine Kolonne wird gleichzeitig ein Azeotrop bestehend aus Isobutylformiat, Ameisensäure und Wasser aus dem Reaktionskolben abdestilliert. Nach Zulaufende wird noch weitere 2 Stunden bei Siedetemperatur nachgerührt und weiter abdestilliert. Dabei steigt die Temperatur auf 115°C an. Der Reaktionskolbeninhalt wird mit 15,5 g (0,228 mol) Natriumformiat neutralisiert und anschließend fraktioniert destilliert. Man erhält 305,4 g L-2-Chlorpropionsäure (D: 0,9 % L: 99,1 %) mit einem Gehalt von 99,9 % (GC) Kp₄₀: 102°C; das entspricht einer Ausbeute von 93,7 % d. Th.

### Beispiel 3

329 g (2,0 mol) L-2-Chlorpropionsäureisobutylester (D: 0,8 % L: 99,2 %) und 230 g (5,0 mol) Ameisensäure (100%ig) werden mit 3,4 g (0,035 mol) Schwefelsäure (100%ig) im Kolben vorgelegt. Innerhalb von 3,5 Stunden wird eine Lösung aus 460 g (10 mol) Ameisensäure (100%ig) und 3,4 g (0,035 mol) Schwefelsäure (100%ig) bei Rückfluß (112°C) zudosiert. Über eine Kolonne wird gleichzeitig ein Azeotrop bestehend aus Isobutylformiat, Ameisensäure und Wasser aus dem Reaktionskolben abdestilliert. Nach Zulaufende wird noch weitere 2 Stunden bei Siedetemperatur nachgerührt und weiter abdestilliert. Dabei steigt die Temperatur auf 116°C an. Der Reaktionskolbeninhalt wird mit 9,52 g (0,14 mol) Natriumformiat neutralisiert und anschließend fraktioniert destilliert. Man erhält 205 g L-2-Chlorpropionsäure (D: 0,9 % L: 99,1 %) mit einem Gehalt von 99,8 % (GC) Kp₃₂: 97°C; das entspricht einer Ausbeute von 94,3 % d. Th.

### Beispiel 4

164,5 g (1,0 mol) L-2-Chlorpropionsäureisobutylester (D: 0,8 % L: 99,2 %) und 333 g (5,0 mol) Essigsäure (90%ig) werden mit 3,68 g (0,036 mol) Schwefelsäure (96%ig) im Kolben vorgelegt. Bei 110°C bis 115°C werden innerhalb von 6 Stunden 666 g (10 mol) wasserhaltige Essigsäure (90 %) zudosiert. Gleichzeitig wird ein Azeotrop bestehend aus Isobutylacetat und Wasser über eine Kolonne aus dem Reaktionskolben abdestilliert. Anschließend wird der Reaktionskolbeninhalt mit 5,9 g (0,072 mol) Natriumacetat neutralisiert und fraktioniert destilliert. Man erhält 100 g L-2-Chlorpropionsäure (D: 1,0 % L: 99,0 %) mit einem Gehalt von 99,8 % (GC) Kp₅₄: 107°C; das entspricht einer Ausbeute von 92,0 % d. Th.

### Beispiel 5

329 g (2,0 mol) L-2-Chlorpropionsäureisobutylester (D: 0,8 % L: 99,2 %) und 625 g (10 mol) Essigsäure (96%ig) werden mit 11 g (0,108 mol) Schwefelsäure (96%ig) im Kolben vorgelegt. BiL 114°C bis 120°C werden innerhalb von 7,5 Stunaen 666 g (10 mol) wasserhaltige Essigsäure (90%ig) zudosiert. Gleichzeitig wird ein Azeotrop bestehend aus Isobutylacetat und Wasser über eine Kolonne aus dem Reaktionskolben abdestilliert. Anschließend wird noch weitere 2,5 Stunden bei 120°C nachgerührt und Azeotrop abdestilliert. Das verbleibende Reaktionsgemisch wird mit 17,7 g (0,216 mol) Natriumacetat neutralisiert und fraktioniert. Man erhält 206,8 g L-2-Chlorpropionsäure (D: 1,0 % L: 99,0 %) mit einem Gehalt von 99,7 % (GC) Kp₄₇: 104°C; das entspricht einer Ausbeute von 95,0 % d. Th.

### Beispiel 6

122,5 g (1 mol) D-2-Chlorpropionsäuremethylester (D: 99 % L: 1 %) und 138 g (3 mol) Ameisensäure (100%ig) werden mit 5,5 g (0,054 mol) Schwefelsäure (96%ig) im Kolben vorgelegt. Innerhalb von 75 Minuten wird eine Lösung aus 300 g (6,5 mol) Ameisensäure (100%ig) und 2,9 g (0,0284 mol) Schwefelsäure (96%ig) bei Rückfluß (102°C) zudosiert. Über eine Kolonne wird gleichzeitig ein Azeotrop bestehend aus Methylformiat, Ameisensäure und Wasser aus dem Reaktionskolben abdestilliert. Nach Zulaufende wird noch eine halbe Stunde bei Siedetemperatur nachgerührt und weiter abdestilliert. Dabei steigt die Temperatur auf 106°C an. Der Reaktionskolbeninhalt wird mit 11,2 g (0,165 mol) Natriumformiat neutralisiert und anschließend fraktioniert destilliert. Man erhält 103 g D-2-Chlorpropionsäure (D: 99 % L: 1 %) mit einem Gehalt von 99,4 % (GC), Kp₆₂: 109°C; das entspricht einer Ausbeute von 94,4 % d. Th.

## Patentansprüche

1. Verfahren zur Herstellung von optisch aktiven 2-Halogenpropionsäuren, dadurch gekennzeichnet, daß man die entsprechenden optisch aktiven 2-Halogenpropionsäurealkylester bei erhöhter Temperatur mit einer Carbonsäure in einer Umacylierungsreaktion unter Bildung der optisch aktiven 2-Halogenpropionsäure und des Carbonsäurealkylesters umsetzt und die erhaltene optisch aktive 2-Halogenpropionsäure aus dem Reaktionsgemisch abtrennt.

2. Verfahren nach Anspruch 1, dadurch gekennzeichnet, daß man die Umsetzung bei Temperaturen von 40°C bis 200°C durchführt.

3. Verfahren nach Anspruch 1, dadurch gekennzeichnet, daß man die erhaltene optisch aktive 2-Halogenpropionsäure in der Weise aus dem Reaktionsgemisch abtrennt, daß man aus dem Reaktionsgemisch den bei der Umacylierung gebildeten Carbonsäurealkylester und gegebenenfalls noch vorhandene Carbonsäure durch Abdestillieren abtrennt und die optisch aktive 2-Halogencarbonsäure als Destillationsrückstand erhält.

4. Verfahren nach Anspruch 1 und 2, dadurch gekennzeichnet, daß man die Umsetzung am Siedepunkt der zur Umacylierung eingesetzten Carbonsäure bzw. des bei der Umacylierung gebildeten Carbonsäurealkylesters durchführt.

5. Verfahren nach Anspruch 1 bis 4, dadurch gekennzeichnet, daß der bei der Umacylierung gebildete Carbonsäurealkylester während der Umsetzung abdestilliert wird.

6. Verfahren nach Anspruch 1 bis 5, dadurch gekennzeichnet, daß man die Umsetzung bei Drücken von 0,1 bis 10 bar durchführt.

7. Verfahren nach Anspruch 1 bis 6, dadurch gekennzeichnet, daß man die erhaltene optisch aktive 2-Halogenpropionsäure in der Weise aus dem nach der Umacylierung anfallenden Reaktionsgemisch isoliert, daß man das Reaktionsgemisch einer fraktionierten Destillation unterzieht.

8. Verfahren nach Anspruch 1 bis 7, dadurch gekennzeichnet, daß man die Carbonsäure im Überschuß verwendet.

9. Verfahren nach Anspruch 1 bis 8, dadurch gekennzeichnet, daß man die Umsetzung in Anwesenheit eines sauren Katalysators durchführt.

10. Verfahren nach Anspruch 1 bis 9, dadurch gekennzeichnet, daß man zusätzlich ein unter den Reaktionsbedingungen inertes Lösungsmittel verwendet.

11. Verfahren nach Anspruch 1 bis 10, dadurch gekennzeichnet, daß man es zur Herstellung von optisch aktiven 2-Chlorpropionsäuren verwendet.

## Claims

1. A process for preparing optically active 2-halopropionic acids, which comprises reacting the corresponding optically active alkyl 2-halopropionates at elevated temperature with a carboxylic acid in a transacylation reaction with formation of the optically active 2-halopropionic acid and the alkyl carboxylate, and separating off the resulting optically active 2-halopropionic acid from the reaction mixture.

2. A process as claimed in claim 1, wherein the reaction is carried out at temperatures of from 40°C to 200°C.

3. A process as claimed in claim 1, wherein the resulting optically active 2-halopropionic acid is separated off from the reaction mixture by distillative removal from the reaction mixture of the alkyl carboxylate formed in the transacylation and any carboxylic acid that may still be present and obtaining the optically active 2-halocarboxylic acid as distillation residue.

4. A process as claimed in claim 1 and 2, wherein the reaction is carried out at the boiling point of the carboxylic acid which is employed for the transacylation or of the alkyl carboxylate formed in the transacylation.

5. A process as claimed in any of claims 1 to 4, wherein the alkyl carboxylate formed in the transacylation is distilled off during the reaction.

6. A process as claimed in any of claims 1 to 5, wherein the reaction is carried out at pressures of from 0.1 to 10 bar.

7. A process as claimed in any of claims 1 to 6, wherein the resulting optically active 2-halopropionic acid is isolated from the reaction mixture which is obtained after the transacylation by subjecting the reaction mixture to a fractional distillation.

8. A process as claimed in any of claims 1 to 7, wherein an excess of carboxylic acid is employed.

9. A process as claimed in any of claims 1 to 8, wherein the reaction is carried out in the presence of an acidic catalyst.

10. A process as claimed in any of claims 1 to 9, wherein additionally a solvent which is inert under the reaction conditions is employed.

11. A process as claimed in any of claims 1 to 10, which is used for preparing optically active 2-chloropropionic acids.

## Revendications

1. Procédé pour la préparation d'acides 2-halogénopropioniques optiquement actifs, caractérisé par le fait qu'on fait réagir les esters alkyliques d'acide 2-halogénopropionique optiquement actifs correspondants à température élevée avec un acide carboxylique dans une réaction de transacylation pour former l'acide 2-halogénopropionique optiquement actif et l'ester alkylique d'acide carboxylique et on sépare du mélange réactionnel l'acide 2-halogénopropionique optiquement actif obtenu.

2. Procédé selon la revendication 1, caractérisé par le fait qu'on effectue la réaction à des températures de 40°C à 200°C.

3. Procédé selon la revendication 1, caractérisé par le fait qu'on sépare l'acide 2-halogénopropionique optiquement actif obtenu du mélange réactionnel de telle façon que l'on sépare par distillation, du mélange réactionnel, l'ester alkylique d'acide carboxylique formé dans la transacylation et l'acide carboxylique éventuellement encore présent, et on obtient l'acide 2-halogénocarboxylique optiquement actif en tant que résidu de distillation.

4. Procédé selon la revendication 1 et 2, caractérisé par le fait qu'on effectue la réaction au point d'ébullition de l'acide carboxylique introduit pour la transacylation ou de l'ester alkylique d'acide carboxylique formé dans la transacylation.

5. Procédé selon l'une des revendications 1 à 4, caractérisé par le fait que l'ester alkylique d'acide carboxylique formé lors de la transacylation est éliminé par distillation pendant la réaction.

6. Procédé selon l'une des revendications 1 à 5, caractérisé par le fait qu'on effectue la réaction à des pressions de 0,1 à 10 bar.

7. Procédé selon l'une des revendications 1 à 6, caractérisé par le fait qu'on isole l'acide 2-halogénopropionique optiquement actif obtenu du mélange réactionnel se formant après la transacylation, en soumettant le mélange réactionnel à une distillation fractionnée.

8. Procédé selon l'une des revendications 1 à 7, caractérisé par le fait qu'on utilise l'acide carboxylique en excès.

9. Procédé selon l'une des revendications 1 à 8, caractérisé par le fait qu'on effectue la réaction en présence d'un catalyseur acide.

10. Procédé selon l'une des revendications 1 à 9, caractérisé par le fait qu'on utilise en outre un solvant inerte dans les conditions de la réaction.

11. Procédé selon l'une des revendications 1 à 10, caractérisé par le fait qu'on l'emploie pour la préparation d'acides 2-chloropropioniques optiquement actifs.
